# EUROPEAN PATENT SPECIFICATION

(11) **EP 4 221 571 B1**
(45) Date of publication and mention of the grant of the patent: **18.11.2026**
(21) Application number: 21795193.8
(22) Date of filing: 28.09.2021
(51) Int. Cl.: A61B 5/00, A61B 5/24, G01N 21/47

(54) **PATHLENGTH RESOLVED CW-LIGHT SOURCE BASED DIFFUSE CORRELATION SPECTROSCOPY**
BAHNLÄNGENAUFGELÖSTE CW-LICHTQUELLENBASIERTE DIFFUSE KORRELATIONSSPEKTROSKOPIE
SPECTROSCOPIE PAR CORRÉLATION DIFFUSE BASÉE SUR UNE SOURCE LUMINEUSE À ONDES ENTRETENUES ET RÉSOLUE EN LONGUEUR D'ONDE

(30) Priority: 01.10.2020 US 202063198181 P; 02.03.2021 US 202163155505 P
(43) Date of publication of application: 09.08.2023
(73) Proprietor: University of South Florida, Tampa, FL 33612-9220 (US)
(72) Inventor: SAFI, Abdul Mohaimen, Tampa, Florida 33613 (US); PARTHASARATHY, Ashwin Bharadwaj, Tampa, Florida 33647 (US); MOKA, Sadhu, Tampa, Florida 33612 (US)
(74) Representative: J A Kemp LLP
(86) International application number: PCT/US2021/052419
(87) International publication number: WO 2022/072353

(56) References cited:
- EP-A1- 0 628 804
- WO-A1-2019/204231
- US-A1- 2019 336 001
- US-B2- 7 061 622
- A.G YODH: "Pulsed diffusing-wave spectroscopy: High resolution through nonlinear optical gating", vol. 42, 1 September 1990 (1990-09-01), Retrieved from the Internet <URL:https://journals.aps.org/prb/abstract/10.1103/PhysRevB.42.4744> [retrieved on 19900901]

## Description

### CROSS-REFERENCE TO RELATED APPLICATIONS

This patent applications claims priority from and benefit of the US Provisional Patent Applications No. 63/198,181 filed on October 01, 2020 and No. 63/155,505 filed on March 02, 2021.

### TECHNICAL FIELD

Embodiments of the invention relate, generally, to a Diffuse Correlation Spectroscopy (DCS) methodology (used, for example, to determine various optical properties of target biological tissue and its hemodynamic perfusion characteristics) and, specifically, the DCS systems and methods that are configured to perform the DCS-type measurements with the use of low-coherence continuous-wave (CW) light sources at levels of light intensities that are substantially lower and with pathlengths through the tissue that are substantially longer than those afforded by the use of, for example, time-domain and/or interferometry-based DCS approaches that are currently employed.

Document US2019/336001 teaches a method for performing a path length-resolved diffuse correlation spectroscopy measurement of a target tissue according to the prior art.

### SUMMARY

Embodiments of the invention provide methodology of acquiring the sought-after DCS-type information representing the target tissue (such as, for example, photon time of flight distribution and pathlength resolved autocorrelation functions) with the use of a DCS-type approach in which the resolution (or separability) of the measurement according to pathlengths of used light through the target tissue is performed simply by adjusting a time-delay between two portions of such used light delivered to two spatially-separated surface areas of the tissue.

According to the idea of the invention, the measurements are carried out with a system that employs a necessarily continuous-wave (CW) light source configured to generate low-coherence light (such as, for example, spatially and/or temporally multi-mode laser diodes, LEDs, in contradistinction with the use of highly-coherent laser sources of the systems of related art) and that can employ an otherwise conventional DCS optical detection system. A skilled artisan having an advantage of this disclosure will readily appreciate that operating the DCS system in the necessarily CW light regime allows the user to necessarily improve the signal-to-noise ratio of the measurement and, therefore, allows the user to carry out a measurement along a light path with longer pathlength through the target tissue than that afforded by the existing methodologies utilizing long-coherence-length pulsed / modulated laser light sources, thereby enhancing measurement sensitivity of the proposed DCS instrument to tissues located at greater depths (for example, deeper in the cortex) as compared with shallower-disposed portions of the tissue that can be reliably measured with existing systems. Furthermore - and in stark contradistinction with existing DCS-type system - the use of the low-coherence length light source provides the user with flexibility of controlling the paths along which light passes through the tissue with pathlength increments defined by (and, if needed, substantially equal to) the coherence length of light generated by such source. Notably, an embodiment of the proposed system can be implemented as an 'add-on' to existing DCS instruments, thereby equipping the systems of related art with an ability (currently missing or at least severely limited) to perform blood flow measurements, for example with pathlength and/or depth sensitivity.

Accordingly, embodiments of the invention provide a method for performing a DCS measurement of a target tissue, which method includes a step of directing a first portion of light (that has a coherence length and that is generated by a necessarily continuous-wave light source) to a first surface area of the target tissue while directing a second portion of the same light to an optical detection system through a delay line. The method further includes a step of receiving light from the first portion of light (after such light has traversed the target tissue) at the second surface area of the tissue, in the form of sample light, and directing this sample light to the optical detection system. Here, the first and second surface areas of the tissue are generally spatially separated from one another by a separation distance. The method additionally includes a step of producing (at the optical detection system) signals that represent interference between the second portion of light and the sample light (which sample light has traversed different paths through the target tissue between the first and second surface areas) while switching between first and second of such different paths only by adjusting a delay in the delay line. (For the purposes of the disclosure, and unless expressly defined otherwise, the term interference is used to conventionally imply, describe, and/or relate to a situation when a combination of two or more electromagnetic waveforms (two or more optical waves, for example) produce a resultant wave in which the displacement is either reinforced or canceled. In other words, the terms interference and interfere and similar terms refer to a phenomenon in which two coherent with each other waves superpose to form a resultant wave of greater, lower, or the same amplitude.)

In at least one implementation of the method, the act of directing the first portion of light to the first surface area includes at least one of: directing the first portion of light having a coherence length of at least 0.05 mm, or at least 0.1 mm, or in specific case between about 0.1 mm and about 0.9 mm; and directing the first portion of light generated by the necessarily continuous-wave light source that is configured to form spatially multi-mode distribution of light it generates. Alternatively or in addition, the switching between first and second of the different paths only by adjusting time delay in the delay line may include switching between the first and second of the different paths with pathlength resolution necessarily defined by the coherence length of light generated by the necessarily CW light source.

Alternatively or in addition, and in substantially any implementation of the method, at least one of the step of directing the first portion of light and the step of directing the second portion of light may include transmitting light through a free space and/or through an optical fiber. Alternatively or in addition, and substantially in any implementation of the method, the act of switching light propagating through the target tissue between the first and second of the different paths through such tissue may include changing a pathlength of the light through the target tissue with an increment substantially equal to the coherence length.

Alternatively or in addition, and substantially in every implementation of the method, the necessarily continuous-wave light source may be configured to generate light having such a degree of coherence that a signal from the signals representing the interference of light at the optical detection system represents interference between the second portion of light and the sample light that has traversed a volume of the target tissue at depths not only 1-3 cm (comparable to the depths teachable by the DCS measurements carried out with methodologies if related art) but also at depths in excess of 3 cm (for example, 3-4 cm, or even deeper than 4 cm in some implementations) below the surface of the tissue. Alternatively or in addition, and practically in every embodiment of the method, the necessarily continuous-wave light source may be configured to generate light having such a degree of coherence that the sample light and the second portion of light interfere with one another at the optical detection system while at least one of the following conditions is satisfied: the sample light has been collected at the second surface portion that is separated from the first surface portion by the fixed separation distance (and at least one case - the separation distance exceeding 2.5 cm; and the sample light represents light from the first portion of light that has traversed a pathlength between the first and second surface areas that is greater than a separation distance (and, in at least one case - of at least 20 cm).

Furthermore, any embodiment of the method may additionally include a step of determining intensity autocorrelation functions and parameters (such absorption, light scattering, flow of moving particle) characterizing tissue traversed by light along each of the different paths through the target tissue with the use of electronic circuitry that is specifically configured to receive said signals from the optical detection system. (In at least one case, such electronic circuitry may be configured as a programmable processor, for example a computer processor.)

In a specific version of the latter case, said the step of determining may include a process of numerical fitting of such intensity autocorrelation functions to a negative exponential function that depends on a wave vector value representing the sample light but, at the same time, is independent from an absorption coefficient of said the target tissue; and/or additionally include a step of determining values of intensities of the sample light for each of the different paths such light traversed through the target tissue.

Alternatively or in addition, and substantially in every implementation, the method may include a step of recording a parameter of a blood flow through the target tissue as a function of a depth of a given path, from the different paths, in the target tissue (and, optionally, producing a report representing such a parameter - for example, in a visually-perceivable fashion). In such a case, the process of recording a parameter of the blood flow may include recording such a parameter at a rate of lower than 20 Hz; or between 20 Hz and 100 Hz; or greater than 100Hz, to name just a few of possibilities.

Alternatively or in addition, and substantially in every embodiment of the method, the method is configured to be devoid of using (that is, without using) a source of pulsed light or a light source configured to generate modulated light. The scope of the invention includes a corresponding DCS measurement system configured to operate such as to implement the method as summarized above.

### BRIEF DESCRIPTION OF THE DRAWINGS

For a more complete understanding of the disclosure, reference is made to the following detailed description and accompanying Drawings, of which:
FIG. 1 illustrates schematically a noninvasive measurement of cerebral blood flow (CBF) waveforms with the use of a conventionally-configured Diffuse Correlation Spectroscopy (DCS) system.
FIG. 2 shows a schematic of an embodiment of the invention configured to effectuate pathlength resolved Diffuse Correlation Spectroscopy (PR-DCS) measurements in absence of pulsed or modulated source of light and without the need to use highly coherent light source.
FIG. 3 depicts an example of the data analysis scheme for the disclosed PR-DCS methodology according to an embodiment of the invention.
FIGS. 4A, 4B, 4C, 4D, and 4E present the results of experimental validations of pathlength resolved Diffuse Correlation Spectroscopy (PR-DCS) methodology carried out according to the idea of the invention.

Generally, the sizes and relative scales of elements in Drawings may be set to be different from actual ones to appropriately facilitate simplicity, clarity, and understanding of the Drawings. For the same reason, not all elements present in one Drawing may necessarily be shown in another. While specific embodiments are illustrated in the figures with the understanding that the disclosure is intended to be illustrative, the invention is defined by the appended claims.

### DETAILED DESCRIPTION

Continued biomedical research requires knowledge of data representing the state of health of a target tissue which, in turn, demands that technologies enabling noninvasive *in vivo* measurements of tissue functions not only possess accuracy and precision, but are also operationally stable while affording repeatable and cost-efficient performance. A skilled artisan knows well that blood flow is one important indicator of tissue health, since characterization of the blood flow directly informs the clinician about the status of oxygen and/or nutrient supply to the target tissue. Therefore, the ability to easily monitor blood flow - and, in particular, outside of clinical environment - has practical significance in treatment of a variety of diseases including strokes, traumatic brain injuries, cancers, and peripheral vascular disorders.

A skilled artisan knows from related art that optical methods based on the diffusion of light through tissue are well suited for noninvasively determining the state of tissue health, for applications ranging from bedside clinical monitoring of physiology to basic science interrogations of tissue function. Optical techniques possess the ability to noninvasively probe a wide variety of tissue types, and they can continuously and safely (optics is non-ionizing) measure biomarkers of tissue health from structures a few centimeters below the surface, without use of any contrast agents. 'Clinical' optical measurement techniques use diffusion of light through tissue to probe tissue vasculature (generally limited to the depths of at most 1-to-3 cm below skin surface) with flexible fiber optic probes placed on the tissue surface. Amongst a broader class of Near Infrared Spectroscopy (NIRS) instruments, the technique of DCS assesses the tissue blood flow from temporal intensity fluctuations of highly coherent light that has diffused through the tissue.

The DCS methodology has rapidly become the method of choice for portable bedside monitoring of deep tissue blood flow. DCS facilitates the sensing and quantification of flow in tissue microvasculature (arterioles) as a blood flow index (BFI). In comparison with other bedside flow monitors, such as Doppler ultrasound, the DCS approach is sensitive to all moving particles in the sampling area, and therefore, measured blood flow is especially well correlated with micro-vascular flow. (The DCS blood flow indices have been validated against a variety of gold standard modalities including Doppler ultrasound and MRI, and its utility has been demonstrated for noninvasive deep tissue measurements in adult/pediatric brain, muscle^{, ,} and spinal cord.)

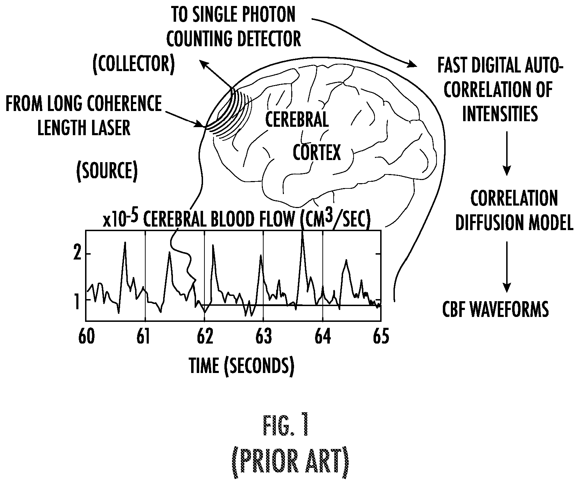

FIG. 1 schematically illustrates a standard, performed according to currently-employed in related art approach DCS measurement (as shown - of the cerebral blood flow waveforms). Here, near infrared light received by the target tissue at a source location from a long coherence length diode laser (such as a 785 nm, 100 mW source produced by Toptica Photonics, for example) is used to illuminate the cerebral cortex using custom fiber optics. Light, multiply scattered upon propagation through the tissue, is collected from the tissue using single mode fiber optics (at a collector location usually 1-4 cm away from the location of the source) and is directed to a single photon counting avalanche photodiode modules (for example, produced by Excelitas SPCM4AQC, Canada) that generate a TTL (transistor-transistor logic) pulse for each of detected photons. Changes in the optical pathlength of light diffusing through the tissue (i.e., due to scattering of moving particles/red blood cells) impart temporal fluctuations in the intensity recorded by the detector of the used optical detection system, which are formally quantified by computation of a digital normalized intensity autocorrelation function *g*₂(*τ*). A quantitative and direct index of blood flow is then estimated (see, for example, Boas, D. A. & Yodh, A. G; in J. Opt. Soc. Am. A 14, 192-215 (1997) or Durduran, T. & Yodh, A. G., in NeuroImage 85 Pt 1, 51-63, doi:10.1016/j.neuroimage.2013.06.017; 2014) by fitting the measured *g*₂(*τ*). to mathematical models appropriate to the measurement geometry (see, e.g., Wang, D. et al., in Biomedical optics express 7, 776-797, doi:10.1364/BOE.7.000776; 2016).. Recently, the digital software processing schemes were employed to increase CBF measurement speeds 100 fold to ~100 Hz, facilitating the first noninvasive optical measurements of pulsatile CBF 'waveforms' (as shown in FIG. 1). (See, for example, Biswas, A. et al., in Clinical and Translational Biophotonics. JW3A. 20 (Optical Society of America); or Tamborini, D. et al., in Neurophotonics 5, 011015, doi:10.1117/1.NPh.5.1.011015;2017). These measurements are similar to those from TCD (transcranial Doppler ultrasound), but they represent perfusion in the cerebral microvasculature, are simpler to measure, and offer enhanced patient comfort. This newfound ability to measure pulsatile CBF dynamics permits a new class of measurements with optics.

However, conventionally-structured DCS methodology suffers from critical limitations that inhibit robust clinical use. DCS - like most optical techniques - is commonly-recognized to be affected by partial volume effects, i.e., the sensitivity of CBF measurement to cerebral tissues is reduced by flow changes in the superficial layers. More specifically, the *g*₂(*τ*). is detected from an ensemble of light pathlengths from different tissue depths - that is, this normalized intensity autocorrelation function is determined as averaged over the multiple pathlengths of light through the tissue Thus - and in reference to the specific application of use of the DCS to assess the cerebral blood flow, as per FIG. 1-the measured blood flow is more sensitive to superficial tissues such as the presence of scalp/skull (which are sampled with higher probability) rather than the brain itself (which are sampled with lower probability). In the past, the present inventors have proposed (see, for example, Baker, W. B. et al. , in Neurophotonics 2, 035004, doi:10.1117/1.NPh.2.3.035004; 2015) simple interventions and multi-layered tissue models to account for these effects - but these remain not necessarily practical for long-term continuous clinical monitoring.

Secondly, as a skilled artisan well knows, conventionally-structured DCS requires *in-situ* measurements of tissue optical properties to improve the fidelity of CBF estimates and facilitate robust comparisons between measurement sites/patients. As a result, conventional DCS instruments are often accompanied and/or complemented with a second, independent and distinct optical instrument to measure tissue optical properties into a hybrid probe. The result of this hybrid arrangement is 2X increase in cost of the overall measurement system (as compared with the conventional DCS apparatus alone), and reduced instrument portability (~ 3X larger probes, limiting regular use in spatially constrained applications like neonates, spine, and critical care). Yet a more significant concern remains the fidelity of the so-performed hybrid optical measurements. Even with collocated sources and detectors, inherent differences in measurement geometry leads to Diffuse Optical Spectroscopy (DOS) and DCS instruments sampling different - not the same! - tissue volumes (see, for example, Binzoni, T. et al. Depth sensitivity of frequency domain optical measurements in diffusive media. Biomed. Opt. Express 8, 2990-3004, doi:10.1364/BOE.8.002990; 2017); or Bevilacqua, F., et al. ; Sampling tissue volumes using frequency-domain photon migration. Phys. Rev. E 69, 051908, doi:10.1103/Phys. Rev. E. 69.051908 ; 2004), thereby inevitably resulting in errors in estimation of blood flow, tissue oxygen saturation, or both. Differences in sampling volume are especially significant in realistic human tissues (e.g., skin) that are heterogeneous. For example, in cerebral measurements, scalp, skull, and brain tissues have significantly different optical properties. As such, DCS and multi- distance DOS) may sample different volume fractions of these tissues. These effects further confound errors of multi-distance measurements of tissue hemodynamics.

Even the recently introduced Time-Domain DCS and interferometric NIRS measurement methodologies while measuring tissue optical properties based on photon diffusion time of flight (DTOF) to improve the fidelity of blood flow measured with DCS, necessarily require the use of specialized pulsed/modulated laser sources with high temporal coherence and speed improvements to traditional DCS detectors in order to attempt to produce pathlength resolved measurements of DCS intensity autocorrelation functions.

In stark and advantageous contradistinction with related art, the present disclosure describes various embodiments of systems and methods for measuring of blood flow with a specifically - in a particular manner - pathlength-resolving Diffuse Correlation Spectroscopy (PR-DCS) instrument configured for depth-sensitive assessment of parameters representing blood flow (such as, for example, pulsatile cerebral blood flow, or CBF). The specific nature of the pathlength resolution provided by the discussed below PR-DCS apparatus manifests in the fact that the spatial increment with which different paths traversed by light through the target tissue subject to the measurement is substantially equal to the coherence length of the necessarily continuous-wave light of the low-coherence light source chosen to be used in such apparatus.

Embodiments of the invention relate, generally, to a diffuse correlation spectroscopy methodology (used, for example, to determine various optical properties of target biological tissue and its hemodynamic perfusion characteristics) and, specifically, the DCS systems and methods that are configured to perform the DCS-type measurements with the use of low-coherence CW light sources at levels of light intensities that are substantially lower and selectively with pathlengths through the tissue that are substantially longer than those afforded by the use of, for example, time-domain and/or interferometry-based DCS approaches that are currently employed.

To this end, persisting problems of inherent instability of operation, associated high levels of measurement noise, and need to use relatively high levels of intensity of light caused by a need to necessarily utilize long coherence length pulsed or at least amplitude-modulated light sources to perform the DCS-type measurements according to methodologies currently used in related art are overcome by configuring a measurement system with a necessarily CW light source (that is, which is necessarily devoid of operation in the pulsed and/or amplitude modulate regime) that has a short-coherence length and/or that is partially coherent. The low level measurement noise floor afforded by such configuration allows the measurement to be performed over longer path lengths through the tissue and/or detection of the target species at lower levels of concentration in the target tissue and/or detection of the target species at greater depths in tissue (in advantageous contradistinction with the existing methodologies).

Furthermore, the use of the low-coherence source in a Mach-Zehnder-type interferometric configuration of an embodiment of the invention enables a practically advantageous - over the methods of related art - flexibility of coherence gating of the light source. (Such coherence gating manifests in causing the measurement to be necessarily pathlength resolved by simply and only adjusting the optical delay between the signal and reference portions of light directed at the target tissue from the low coherence source of light.) Moreover, the use of the proposed methodology manifests in avoiding altogether - in contradistinction with the methodologies of related art - the complementary (and, conventionally near-infrared spectroscopy, or NIRS measurement) to be performed with a separate, independent instrument to obtain the in-situ high-fidelity measurement data.

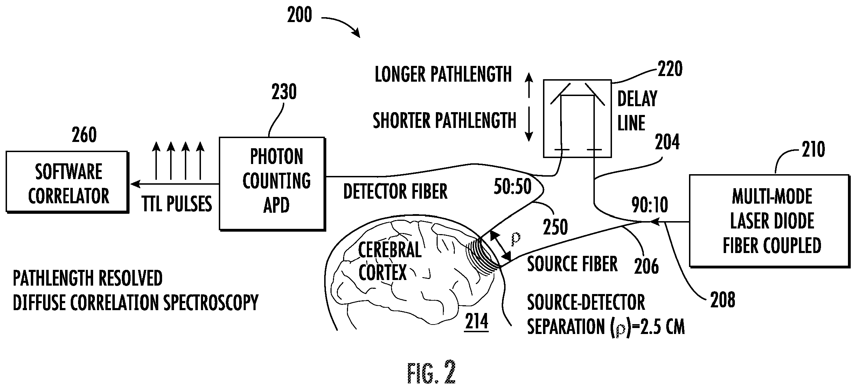

In various embodiments of the implementation of the idea of the current invention, the pathlength resolved measurement of the target species of the tissue (for example, the CBF flow) turns on coherence gating - implemented with the use of a delay line configured (at least in the example of FIG. 2) as a Mach-Zehnder interferometer. FIG. 2 provides a schematic 200 of PR-DCS methodology configured according to the idea of the invention, in which corresponding portions 204 and 206 of the light output 208 from a necessarily CW (and, optionally, a spatially multi-mode and/or spectrally broadband) light source 210 are directed to a programmable delay line 220 (and though it - towards the optical detection system 230) and to the sample (target tissue) 214 (and through it - towards the optical detection system 230), respectively. In the example of FIG. 2, the delay line 220 is disposed in a reference arm of the set-up configured as a Mach-Zehnder interferometer. A standard DCS detector (optical detection) arrangement or system 230 can be used to record the heterodyne interference between the optical signal arriving to the optical detection system 230 along the sample arm of the employed interferometric arrangement (which incorporates the sample 214) and that arriving along the reference arm (which incorporates the delay line 220). Depending on the specifics of a particular implementation, light portions propagating through the sample and reference arms of the apparatus can be delivered through free space, optical fibers, or both - to name just a few of possibilities.

A typical off-the-shelf continuous wave partially coherent laser diode (such as, for example, L808P0500MM, Thorlabs, NJ) featuring a nominal coherence length of from about 0.1 mm to about 0.2 mm or so (or at least 0.1 mm; in one example 0.5 mm, as measured with high- resolution spectrometer) can be employed as a light source 210. As shown in this specific example 200, one portion of the light output the laser diode 210 is coupled to a 90: 10 multi-mode fiber coupler; the distal end of the 90% arm (sample arm) optical fiber channeling light 206 is directed to the tissue surface, while the 10% arm (reference arm) optical fiber channeling light 204 is directed to a combination of reflectors forming the variable delay line 220 mounted on a translation stage. It is understood that, generally, the delay line 220 is a device producing a specific desired delay in the transmission of a signal through such line. Light collected from the tissue (as shown - at a location separated by a distance ρ about or greater than 2.5 cm away from the location at which light portion 206 was delivered to the tissue) is collected with another single-mode fiber element 250, recombined (99:1 ratio) with a portion of light delivered from the delay line 220, and directed to the optical detection system 230. A custom software correlator device 260 (which may optionally be implemented in LabVIEW with the use of appropriate programmable electronic circuitry) is configured to compute the intensity autocorrelation functions in real-time at various measurement rates - for example, at 20~100 Hz.

The employed DCS detector system 230 is configured to measure the mixture of electric fields arriving from the sample arm (*E_{S}*(*t*)) and the reference arm ( *E_{R}*(*t*))) arms, i.e., to produce a signal representing the optical intensity $I \left(t\right) = {\left|{E}_{S}\right|}^{2} + {\left|{E}_{R}\right|}^{2} + {E}_{S} {E}_{R}^{*} + {E}_{R} {E}_{S}^{*}$, where *E_{S}* represents the superposition of sample light fields that have travelled multiple pathlengths through the tissue and *E_{R}* represents the reference light field arriving to the system 230 from the delay line 220. Understandably, coherent interference of the sample and reference fields occurs only between fields that have traveled the same pathlength - thus, pathlength specific detection is necessarily achieved in the embodiment of the invention by simply varying (and in one case - varying only) the length of the reference arm - in one case, by varying the amount of delay introduced by the delay line 220. The intensity detected at 230 can be approximated as *I*(*s, t*) *= I*₀ *+ I_{DCS}*(*s, t*), where *I*₀ is an incoherent addition of light fields arriving from the sample and reference arms that are not pathlength matched. *I*₀ is a constant (approximately) that can be directly measured as the sum of the average intensities with the sample and reference arms blocked (i.e. under conditions *E_{S}* = 0, *E_{R}* = 0 ). When *E_{S}* = 0 , *I*(*t*) = |*E_{R}*|² and when *E_{R}* = 0 *I*(*t*) = |*E_{S}*|² both of which conditions represent incoherent addition of the respective light fields; therefore, *I*₀ ≈ |*E_{S}*|² + |*E_{R}*|²*. I_{DCS}*(*s, t*) is the (heterodyne) pathlength-matched interference term with temporal intensity fluctuations brought about by moving particles in the sample, around/about the selected pathlength and within the coherence length of the laser. Autocorrelation of *I*(*t*)yields the pathlength-resolved intensity autocorrelation function *g*₂(*τ, s*) = 1 + *β*(*s*)|*g*₁(*τ,s*)|², where *β*(*s*)is the pathlength-dependent instrumentation/speckle averaging factor, τ is the correlation delay time, S is the light pathlength through the target tissue, and *g*₁(*τ, s*) is the pathlength-resolved electric field autocorrelation function. Notably, the resolution between different pathlengths of light in the sample arm through the target tissue that can be detected and implemented in an embodiment is defined by (and is substantially equal to) the coherence length of light produced by the CW source 210. Fitting *g*₁(*τ, s*) to a negative exponential model, ${g}_{1} \left(τ, s\right) = exp \left(-2{μ}_{s}^{′}{Fk}_{0}^{2}sτ\right)$*,* yields estimates of blood flow (F). Here, ${μ}_{s}^{′}$ is the tissue reduced scattering coefficient and k₀ is the wave vector of used light. Note that *g*₁(*τ, s*) is dependent only on F and ${μ}_{s}^{′}$ and is independent from the tissue absorption coefficient *µₐ* . Since ${μ}_{s}^{′}$ is typically constant in the tissue, pathlength-resolved measurements of relative blood flow changes are **substantially** insensitive to baseline tissue optical properties. Furthermore, the average of the measured intensity *I_{DCS}*(*s, t*) = 〈*I_{DCS}*(*s, t*)〉 can be further converted to time-resolved diffuse reflectance data by recognizing that photon time-of-flight in target tissue is δt = s/v, where v denotes the speed of light in the tissue. Thus, *I_{DCS}*(*s*) → *I_{DCS}*(*t*), which - when appropriately normalized - yields diffusion time-of-flight curves that can be fit to a time-domain solution of the photon diffusion equation (see, for example, Durduran, T., et al. ; Diffuse optics for tissue monitoring and tomography, Reports on progress in physics 73, 076701, doi:10.1088/0034-4885/73/7/076701; 2010) to estimate tissue optical properties, *µₐ* and ${μ}_{s}^{′}$.

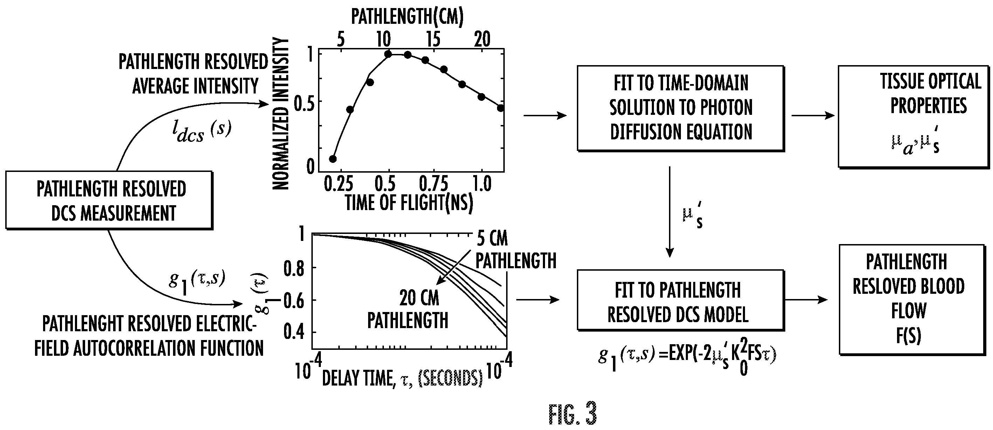

FIG. 3 schematically illustrates an example of the data analysis flow for the embodiment of the proposed PR-DCS methodology, as discussed above Accordingly, with the use of an embodiment of the invention, a single measurement - not a combination of two measurements required by related art - yields both the pathlength-resolved average intensities (top row of FIG. 3, used to estimate optical properties of the tissue) and electric field autocorrelation functions (bottom row of FIG. 3, used to estimate moving particles in the tissue - for example blood flow). Once optical properties are computed, fast CBF measurements can be performed at a specific set/desired pathlength of light (in the sample arm of the embodiment of FIG. 2) through the tissue 214. Notably, relative CBF measurements are independent from optical properties, since ${μ}_{s}^{′}$ is a constant. As such, FIG. 3 presents an example of data processing protocol for fast, *in vivo,* depth-sensitive measurements of blood flow, in which the DCS intensity autocorrelation functions and average intensities for multiple pathlengths are simultaneously. For a spatial separation, along the surface of the target tissue 214, between the area at which the light in the sample arm is used to irradiate the tissue and the area from which the sample light is collected from the tissue, of , for example, 2.5 cm, these measurements span the range of pathlengths from about 5 cm to about 30 cm. Light propagating through the tissue along paths with pathlengths in excess of 20 cm selectively, with the pathlength resolution defined by the coherence length of employed light, samples the volumes of the tissue located as deep as 3 cm to 4 cm below the tissue surface, which is not achievable by related art employing the highly coherent light source . (As was already alluded to above, pathlength resolved average intensities can be further fit to a time-domain photon diffusion solution to first estimate tissue optical properties (*µₐ* and ${μ}_{s}^{′}$), while the pathlength-resolved intensity autocorrelation functions are used to estimate flow as a function of pathlength/tissue depth). Then, the reference arm can be fixed with a desired pathlength (depending on the tissue depth of interest), to record high-speed (for example, at 20~100 Hz) depth-sensitive changes in blood flow.

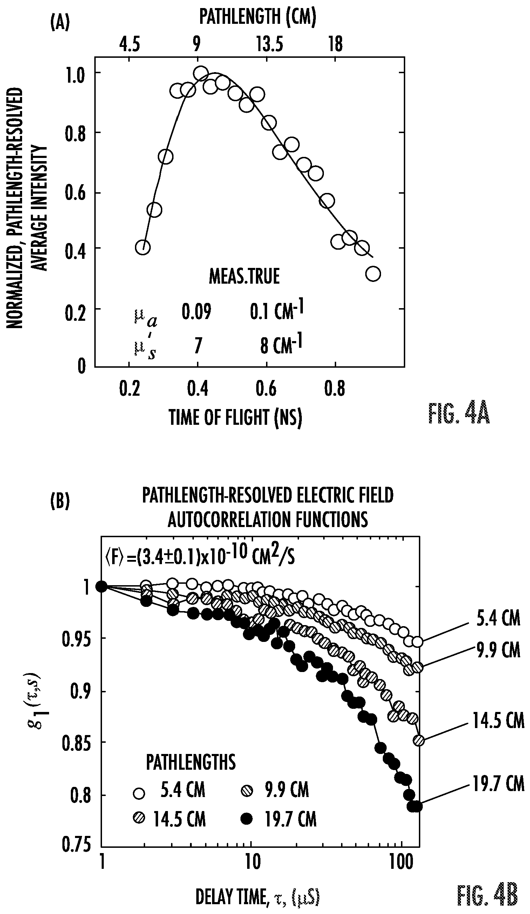

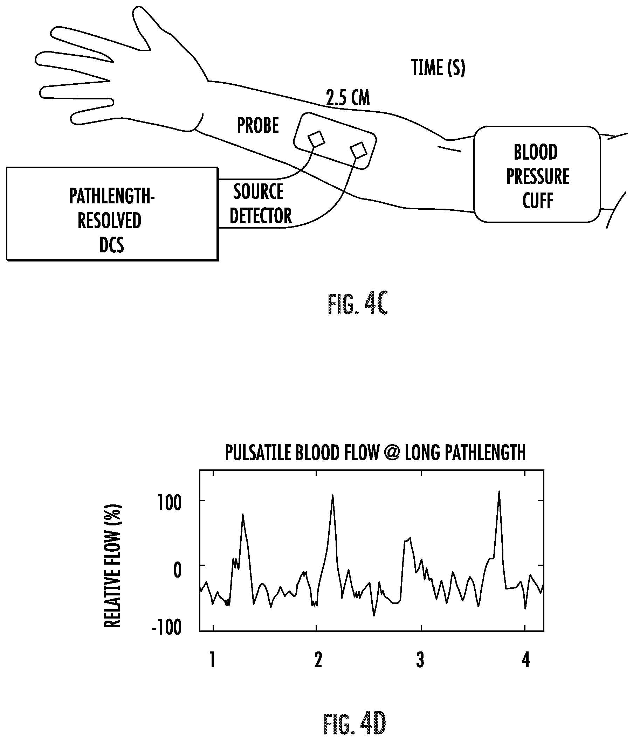

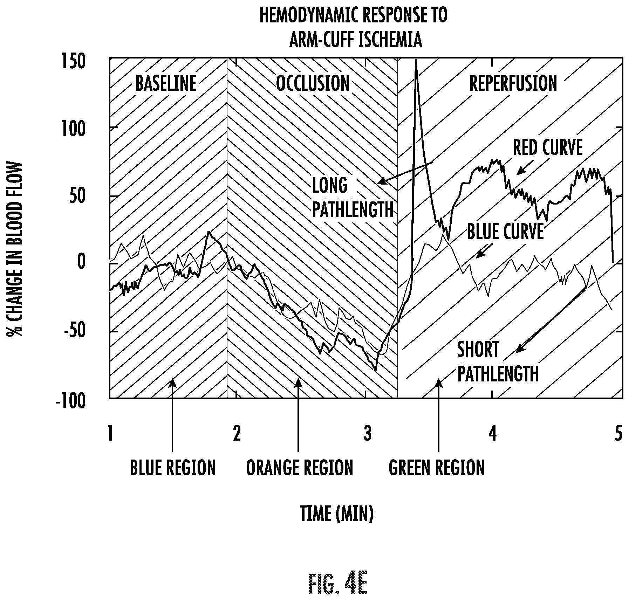

FIGs. 4A, 4B, 4C, 4D, and 4E present some experimental validation of the use of an embodiment of Pathlength Resolved Diffuse Correlation Spectroscopy methodology discussed above, demonstrating both the feasibility and workability of pathlength-resolved blood flow measurements by estimating the static (as shown in FIG. 4A) and dynamic (as shown in FIG. 4B) optical properties of a semi-infinite tissue-simulating phantom (made of intralipid and India ink, with known values of *µₐ* = 0.1 cm⁻¹ and ${μ}_{s}^{′} = 8 {cm}^{- 1}$). Per the idea of the invention, the PR-DCS measurements were performed with a source-detector separation of ρ =2.5 cm and integration time of 1 second.

Specifically, FIG. 4A displays the diffusion time-of-flight (DTOF) curve obtained by an analysis of pathlength-resolved average intensities (indicated with circles) fit to a photon diffusion model (a time-domain solution to the diffusion equation; black line), such that optical properties (*µₐ*= 0.09 cm⁻¹, ${μ}_{s}^{′} = 7 {cm}^{- 1}$) of this homogeneous tissue-simulated phantom, which were recovered to within 10% of their true values.

FIG. 4B shows representative pathlength-resolved electric field autocorrelation functions, assessed with an embodiment of the invention as discussed above and fit to a DCS negative exponential model to estimate blood flow. Blood flow index, (F) = (3.4 ± 0.1) × 10⁻¹⁰cm²/s (in units of diffusion), was found to be substantially constant (within about 3%) across a multiplicity of different pathlengths, as a skilled artisan should expect in this homogenous semi-infinite tissue phantom.

The schematic presented in FIG. 4C demonstrates the ability of the proposed methodology to measure depth-sensitive blood flow with an *in vivo* arm cuff ischemia experiment. Here, the PR-DCS measurements were performed on an arm of a healthy volunteer with a source-detector separation ρ of at least 2.5 cm and at light pathlengths of 5.4 cm (short) and 20.5 cm (long) and longer.

FIG. 4D presents a representative time course of pulsatile blood flow measured in the arm of a volunteer with the embodiment of PR-DCS at a 20.5 cm pathlength and a 20 Hz blood flow measurement rate. Based on assessment of FIG. 4D, a skilled person will appreciate that the blood flow pulsatility is clearly resolved, thereby demonstrating a high signal-to-noise ratio of the proposed approach. To the inventors' knowledge, this demonstration was the first pulsatile pathlength-resolved blood flow measurement with DCS.

Additionally, FIG. 4E illustrates relative changes in arm blood flow during arm-cuff ischemia measured at 1Hz with both long and short pathlengths for 1-minute baseline (represented by "blue region" of the plot), an approximately 2-minute occlusion (represented by the "orange region" of the plot), and 2-minute recovery (represented by the "green region" of the plot). Here, red and blue curves respectively indicate flow changes at 20.5 and 5.4 cm pathlengths respectively. Hemodynamic response and reperfusion peak observed at the long pathlength (red curve) is typical of a response from larger blood vessels in the muscle that lie deeper within the tissue. On the other hand, the corresponding response observed at the shorter pathlength from smaller, shallower blood vessels is stunted, which indicates that the tissue sampled is primarily microvasculature in the skin. These preliminary experiments clearly demonstrate that the embodiment of the PR-DCS instrument is sufficiently robust to perform reliable measurements of static and dynamic tissue optical properties. In addition, the signal-to-noise ratio of the measurements is high enough to observe pulsatile blood flow changes even at longer pathlengths (low probability events), and the instrument can clearly discriminate between superficial and deep-tissue blood flow changes.

The skilled artisan will readily appreciate - and it is worth reiterating - that advantages of the proposed PR-DCS apparatus and method over other instruments that may be targeting pathlength resolved measurements include the fact that the proposed methodology is based on the use of a light source operating in a continuous-wave regime, and that the spatial resolution between different paths through the tissue (along which paths the portion of light from such light source traverses the tissue) is seamlessly controlled with increments defined by the coherence length of such light. Therefore, with the use of an embodiment of the proposed idea there is not photon budget penalty to be paid for measuring long pathlength photons that require large spatial source-detector separations (see ρ, FIG. 2), and the detection probabilities of the target species in the tissue are high, whereas most time domain-DCS (TD-DCS) instruments are limited to a source-detector separation of about 1 cm (which shortcoming is well recognized in related art, as evidenced, for example, by Sutin, J. et al. Time-domain diffuse correlation spectroscopy. Optica 3, 1006-1013, doi:10.1364/OPTICA.3.001006,2016; and Pagliazzi, M. et al. Time domain diffuse correlation spectroscopy with a high coherence pulsed source: in vivo and phantom results. Biomedical Optics Express 8, 5311-5325, doi:10.1364/BOE.8.005311,2017).

With the CW operation of the measurement system, and the resulting measurement is substantially Shott noise-limited, thereby permitting long-separation distances ρ and long-pathlength measurements to be performed at lower laser powers as compared with those employed in related art. Clearly, therefore, deeper tissues *in vivo* can be sampled with the PR-DCS than those reachable with currently-existing technology. Furthermore, as a corollary to the use of the CW light source, the measurement system understandably does not require periodic calibrations/corrections based on instrument response, jitter compensations, or phase stabilizations, for example (which are common-use in related art). Finally, practically any off-the-shelf, easy to use, low-cost CW laser diode and standard DCS detector can be used to construct the measurement system according to the idea of the invention. In stark contradistinction to the advantages offered by the disclosed PR-DCS, TD-DCS requires specialized high (long) coherence length pulsed laser sources (and simply cannot be implemented with the CW sources) and high sensitivity, red-enhanced single-photon avalanche diode (SPAD) detectors, while interferometric NIRS requires frequency swept lasers, balanced detectors, and electronics that operate in the GHz range.

For the purposes of this disclosure and the appended claims, the use of the terms "substantially", "approximately", "about" and similar terms in reference to a descriptor of a value, element, property or characteristic at hand is intended to emphasize that the value, element, property, or characteristic referred to, while not necessarily being exactly as stated, would nevertheless be considered, for practical purposes, as stated by a person of skill in the art. These terms, as applied to a specified characteristic or quality descriptor means "mostly", "mainly", "considerably", "by and large", "essentially", "to great or significant extent", "largely but not necessarily wholly the same" such as to reasonably denote language of approximation and describe the specified characteristic or descriptor so that its scope would be understood by a person of ordinary skill in the art. In one specific case, the terms "approximately", "substantially", and "about", when used in reference to a numerical value, represent a range of plus or minus 20% with respect to the specified value, more preferably plus or minus 10%, even more preferably plus or minus 5%, most preferably plus or minus 2% with respect to the specified value. As a non-limiting example, two values being "substantially equal" to one another implies that the difference between the two values may be within the range of +/- 20% of the value itself, preferably within the +/- 10% range of the value itself, more preferably within the range of +/- 5% of the value itself, and even more preferably within the range of +/- 2% or less of the value itself. The use of these terms in describing a chosen characteristic or concept neither implies nor provides any basis for indefiniteness and for adding a numerical limitation to the specified characteristic or descriptor. As understood by a skilled artisan, the practical deviation of the exact value or characteristic of such value, element, or property from that stated falls and may vary within a numerical range defined by an experimental measurement error that is typical when using a measurement method accepted in the art for such purposes.

The skilled artisan can now readily appreciate that embodiments of the invention address the operational problems persisting with the use of related art methodology by employing a new PR-DCS approach configured to effectuate depth-sensitive measurements the target tissue (and, in particular - of pulsatile CBF in humans). PR-DCS employs a continuous-wave low coherence interferometer that can be configured to measure CBF changes from tissue depths of at least 3~4 cm below the surface - sufficient to be sensitive to the entire cerebral cortex and even some sub-cortical structures. Critically, in various embodiments, PR-DCS realizes these CBF measurements with readily available laser diodes and conventional DCS detection systems, as it does not require the use of pulsed lasers, frequency-modulated light sources, or custom detector configurations typically used in other pathlength resolved instruments of related art.

While the invention is described through the above-described exemplary embodiments, it will be understood by those of ordinary skill in the art that modifications to, and variations of, the illustrated embodiments may be made and that the invention is defined by the appended claims.

## Claims

1. A method for performing a pathlength-resolved diffuse correlation spectroscopy (PR-DCS) measurement of a target tissue (214), the method comprising:
directing a first portion of light that has a coherence length and that is generated by a necessarily continuous-wave light source (210) to a first surface area of the target tissue while directing a second portion of light generated by said light source to an optical detection system (230) through a delay line (220);
receiving light from the first portion of light, that has traversed the tissue, at the second surface area of the tissue as a sample light and directing said sample light to the optical detection system through a single optical channel,
wherein the first and second surface areas of the tissue are spatially separated from one another by a separation distance; and
at the optical detection system comprising a single photodetector, producing signals representing interference between the second portion of light transmitted through said single optical channel and the sample light that has traversed different paths through the target tissue between the first and second surface areas and transmitted through said single optical channel while switching between first and second of said different paths only by adjusting a delay in the delay line, wherein said necessarily continuous-wave light source is configured to generate light having such a degree of coherence that the sample light and the second portion of light interfere with one another at the optical detection system while the following conditions are satisfied:
(a) the sample light is produced at the second surface portion that is separated from the first surface portion by the separation distance exceeding 2.5 cm; and/or
(b) the sample light represents said light from the first portion of light that has traversed a pathlength greater than the separation distance.

2. A method according to claim 1, wherein the directing the first portion of light to the first surface area includes:
(2a) directing the first portion of light having a coherence length of at least 0.05 mm; and/or
(2b) directing the first portion of light generated by the necessarily continuous-wave light source configured to form spatially multi-mode distribution of generated light; and/or
(2c) the coherence length becomes the pathlength resolution of the measurement.

3. A method according to claim 2, wherein the switching between first and second of the different paths only by adjusting time delay in the delay line includes switching between said first and second of the different paths with pathlength resolution defined by the coherence length.

4. A method according to one of claims 1, 2, and 3, wherein at least one of said directing the first portion of light and said directing the second portion of light through a single channel includes transmitting light through free space or through an optical fiber towards the single optical detector.

5. A method according to one of claims 1 through 4, wherein the switching between the first and second of said different paths includes changing a pathlength of the sample light through the target tissue with an increment substantially equal to the coherence length.

6. A method according to one of claims 1 through 5, wherein said necessarily continuous-wave light source is configured to generate light having such a degree of coherence that a signal from the signals representing said interference and produced at the optical detection system represents interference between the second portion of light and the sample light that has traversed a volume of the target tissue at a depth of at least 1 cm below a surface of the tissue.

7. A method according to one of claims 1 through 6, further comprising determining intensity autocorrelation functions and parameters characterizing tissue traversed by each of the different paths through the target tissue with the use of electronic circuitry configured to receive said signals from the optical detection system.

8. A method according to claim 7, wherein said determining includes numerical fitting of said intensity autocorrelation functions to a negative exponential function that depends from a wave vector value representing said sample light but does is independent from an absorption coefficient of said target tissue.

9. A method according to one of claims 7 and 8, further comprising determining values of intensities of the sample light for each of the different paths.

10. A method according to one of claims 1 through 9, further comprising:
recording a parameter of a blood flow through the target tissue as a function of a depth of a given path, from the different paths, in the target tissue.

11. A method according to claim 10, wherein the recording a parameter of the blood flow includes recording said parameter at a rate of lower than 20 Hz.

12. A method according to claim 10, wherein the recording a parameter of the blood flow includes recording said parameter at a rate of between 20 Hz and 100 Hz.

13. A method according to claim 10, wherein the recording a parameter of the blood flow includes recording said parameter at a rate of greater than 100 Hz.

14. A method according to one of claims 1 to 13, that is devoid of using a source of pulsed light or a light source configured to generate modulated light.

## Patentansprüche

1. Verfahren zum Durchführen einer weglängenaufgelösten diffusen Korrelationsspektroskopie(PR-DCS)-Messung eines Zielgewebes (214), wobei das Verfahren umfasst:
Richten eines ersten Lichtanteils, der eine Kohärenzlänge aufweist und der von einer zwangsläufigen Dauerstrichlichtquelle (210) erzeugt wird, auf einen ersten Oberflächenbereich des Zielgewebes, während ein zweiter, von der Lichtquelle erzeugter Lichtanteil auf ein optisches Erkennungssystem (230) durch eine Verzögerungsleitung (220) gerichtet wird;
Empfangen von Licht von dem ersten Lichtanteil, das das Gewebe durchlaufen hat, an dem zweiten Oberflächenbereich des Gewebes als ein Probenlicht und Richten des Probenlichts durch einen einzigen optischen Kanal auf das optische Erkennungssystem,
wobei der erste und der zweite Oberflächenbereich des Gewebes räumlich voneinander durch einen Trennabstand getrennt sind; und
in dem optischen Erkennungssystem, das einen einzelnen Fotodetektor umfasst, Produzieren von Signalen, die die Interferenz zwischen dem zweiten Lichtanteil, der durch den einzelnen optischen Kanal übertragen wird, und dem Probenlicht, das unterschiedliche Wege durch das Zielgewebe zwischen dem ersten und dem zweiten Oberflächenbereich durchlaufen hat und durch den einzigen optischen Kanal übertragen wird, während zwischen dem ersten und dem zweiten der unterschiedlichen Wege ausschließlich durch Anpassen einer Verzögerung in der Verzögerungsleitung umgeschaltet wird, wobei die zwangsläufige Dauerstrichlichtquelle dazu konfiguriert ist, Licht mit einem solchen Kohärenzgrad zu erzeugen, dass das Probenlicht und der zweite Lichtanteil in dem optischen Erkennungssystem einander stören, während die folgenden Bedingungen erfüllt sind:
(a) das Probenlicht wird an dem zweiten Oberflächenbereich erzeugt, der von dem ersten Oberflächenbereich durch einen Trennabstand von mehr als 2,5 cm getrennt ist; und/oder
(b) das Probenlicht stellt das Licht aus dem ersten Lichtanteil dar, das eine Weglänge zurückgelegt hat, die größer ist als der Trennabstand.

2. Verfahren nach Anspruch 1, wobei das Richten des ersten Lichtanteils auf den ersten Oberflächenbereich beinhaltet:
(2a) Richten des ersten Lichtanteils mit einer Kohärenzlänge von wenigstens 0,05 mm; und/oder
(2b) Richten des ersten Lichtanteils, der durch die zwangsläufige Dauerstrichlichtquelle erzeugt wird und dazu konfiguriert ist, eine räumlich multimodale Verteilung des erzeugten Lichts zu erzeugen; und/oder
(2c) die Kohärenzlänge wird zu der Weglängenauflösung der Messung.

3. Verfahren nach Anspruch 2, wobei das Umschalten zwischen einem ersten und einem zweiten der unterschiedlichen Wege ausschließlich durch Anpassen der Zeitverzögerung in der Verzögerungsleitung das Umschalten zwischen dem ersten und dem zweiten der unterschiedlichen Wege mit einer durch die Kohärenzlänge definierten Weglängenauflösung beinhaltet.

4. Verfahren nach einem der Ansprüche 1, 2 und 3, wobei wenigstens eines des Richtens des ersten Lichtanteils und des Richtens des zweiten Lichtanteils durch einen einzigen Kanal das Übertragen von Licht durch den freien Raum oder durch eine optische Faser in Richtung des einzigen optischen Detektors beinhaltet.

5. Verfahren nach einem der Ansprüche 1 bis einschließlich 4, wobei das Umschalten zwischen dem ersten und dem zweiten der unterschiedlichen Wege das Ändern einer Weglänge des Probenlichts durch das Zielgewebe mit einem Inkrement im Wesentlichen gleich der Kohärenzlänge beinhaltet.

6. Verfahren nach einem der Ansprüche 1 bis einschließlich 5, wobei die notwendige Dauerstrichlichtquelle dazu konfiguriert ist, Licht mit einem solchen Kohärenzgrad zu erzeugen, dass ein Signal aus den Signalen, die die Interferenz darstellen und an dem optischen Erkennungssystem produziert werden, die Interferenz zwischen dem zweiten Lichtanteil und dem Probenlicht darstellt, das ein Volumen des Zielgewebes in einer Tiefe von wenigstens 1 cm unterhalb einer Oberfläche des Gewebes durchlaufen hat.

7. Verfahren nach einem der Ansprüche 1 bis einschließlich 6, das ferner das Bestimmen von Intensitätsautokorrelationsfunktionen und Parametern beinhaltet, die das von jedem der unterschiedlichen Wege durch das Zielgewebe durchquerte Gewebe kennzeichnen, unter Verwendung einer Elektronikschaltung, die dazu konfiguriert ist, die Signale von dem optischen Erkennungssystem zu empfangen.

8. Verfahren nach Anspruch 7, wobei das Bestimmen das numerische Anpassen der Intensitätsautokorrelationsfunktionen an eine negative Exponentialfunktion beinhaltet, die von einem Wellenvektorwert abhängig ist, der das Probenlicht darstellt, jedoch von einem Absorptionskoeffizienten des Zielgewebes unabhängig ist.

9. Verfahren nach einem der Ansprüche 7 und 8, das ferner das Bestimmen von Werten der Intensitäten des Probenlichts für jeden der unterschiedlichen Wege umfasst.

10. Verfahren nach einem der Ansprüche 1 bis einschließlich 9, das ferner umfasst:
Aufzeichnen eines Parameters eines Blutflusses durch das Zielgewebe abhängig von einer Tiefe eine gegebenen Wegs von den unterschiedlichen Wegen in dem Zielgewebe.

11. Verfahren nach Anspruch 10, wobei das Aufzeichnen eines Parameters des Blutflusses das Aufzeichnen des Parameters mit einer Rate von weniger als 20 Hz beinhaltet.

12. Verfahren nach Anspruch 10, wobei das Aufzeichnen eines Parameters des Blutflusses das Aufzeichnen des Parameters mit einer Rate von zwischen 20 Hz und 100 Hz beinhaltet.

13. Verfahren nach Anspruch 10, wobei das Aufzeichnen eines Parameters des Blutflusses das Aufzeichnen des Parameters mit einer Rate von mehr als 100 Hz beinhaltet.

14. Verfahren nach einem der Ansprüche 1 bis 13, wobei keine Quelle von gepulstem Licht oder eine Lichtquelle verwendet wird, die dazu konfiguriert ist, moduliertes Licht zu erzeugen.

## Revendications

1. Procédé permettant de réaliser une mesure par spectroscopie de corrélation diffuse à résolution en longueur de trajet (PR-DCS) d'un tissu cible (214), le procédé comprenant :
le guidage d'une première portion de lumière, présentant une longueur de cohérence et générée par une source de lumière (210) nécessairement à onde continue, vers une première région de surface du tissu cible, tandis que s'effectue le guidage d'une deuxième portion de lumière générée par ladite source de lumière vers un système de détection optique (230), dans une ligne à retard (220) ;
la réception de la lumière provenant de la première portion de lumière, et ayant traversé le tissu, au niveau de la deuxième région de surface du tissu en tant que lumière échantillon, et le guidage de ladite lumière échantillon vers le système de détection optique, dans un canal optique unique,
les première et deuxième régions de surface du tissu étant spatialement séparées l'une de l'autre par une distance de séparation ; et
au niveau du système de détection optique, qui comprend un photodétecteur unique, la production de signaux représentant une interférence entre la deuxième portion de lumière transmise dans ledit canal optique unique et la lumière échantillon ayant parcouru différents trajets à travers le tissu cible entre les première et deuxième régions de surface et ayant été transmise dans ledit canal optique unique, tandis que s'effectue un basculement entre un premier et un deuxième desdits différents trajets par seul ajustement d'un retard dans la ligne à retard, ladite source de lumière nécessairement à onde continue étant conçue pour générer une lumière présentant un degré de cohérence tel que la lumière échantillon et la deuxième portion de lumière interfèrent l'une avec l'autre au niveau du système de détection optique, sous les conditions suivantes :
(a) la lumière échantillon est produite au niveau de la deuxième portion de surface qui est séparée de la première portion de surface par une distance de séparation supérieure à 2,5 cm, et/ou
(b) la lumière échantillon représente ladite lumière provenant de la première portion de lumière et ayant parcouru une longueur de trajet supérieure à la distance de séparation.

2. Procédé selon la revendication 1, dans lequel le guidage de la première portion de lumière vers la première région de surface comprend :
(2a) le guidage de la première portion de lumière présentant une longueur de cohérence d'au moins 0,05 mm, et/ou
(2b) le guidage de la première portion de lumière générée par la source de lumière nécessairement à onde continue conçue pour former une distribution spatiale multimode de la lumière générée, et/ou
(2c) la longueur de cohérence devient la résolution en longueur de trajet de la mesure.

3. Procédé selon la revendication 2, dans lequel le basculement entre le premier et le deuxième des différents trajets par seul ajustement d'un retard dans la ligne à retard comprend un basculement entre lesdits premier et deuxième des différents trajets avec une résolution en longueur de trajet définie par la longueur de cohérence.

4. Procédé selon l'une quelconque des revendications 1, 2 et 3, dans lequel au moins un desdits guidages de la première portion de lumière et de la deuxième portion de lumière dans un canal unique comprend la transmission de la lumière dans l'espace libre ou par fibre optique vers le détecteur optique unique.

5. Procédé selon l'une des revendications 1 à 4, dans lequel le basculement entre les premier et deuxième desdits trajets différents comprend la modification de la longueur de trajet de la lumière échantillon à travers le tissu cible d'un incrément sensiblement égal à la longueur de cohérence.

6. Procédé selon l'une quelconque des revendications 1 à 5, dans lequel ladite source de lumière nécessairement à onde continue est conçue pour générer une lumière présentant un degré de cohérence tel qu'un signal, parmi les signaux représentant ladite interférence qui sont produits au niveau du système de détection optique, représente l'interférence entre la deuxième portion de lumière et la lumière échantillon ayant traversé un volume du tissu cible à une profondeur d'au moins 1 cm sous la surface du tissu.

7. Procédé selon l'une quelconque des revendications 1 à 6, comprenant en outre la détermination de fonctions d'autocorrélation d'intensité et de paramètres caractérisant le tissu traversé par chacun des différents trajets, dans le tissu cible, au moyen d'un circuit électronique conçu pour recevoir lesdits signaux provenant du système de détection optique.

8. Procédé selon la revendication 7, dans lequel ladite détermination comprend l'ajustement numérique desdites fonctions d'autocorrélation d'intensité à une fonction exponentielle négative qui dépend d'une valeur de vecteur d'onde représentant ladite lumière échantillon, mais qui est indépendante d'un coefficient d'absorption dudit tissu cible.

9. Procédé selon l'une quelconque des revendications 7 et 8, comprenant en outre la détermination des valeurs d'intensité de la lumière échantillon pour chacun des différents trajets.

10. Procédé selon l'une quelconque des revendications 1 à 9, comprenant en outre :
l'enregistrement d'un paramètre du débit sanguin à travers le tissu cible en fonction de la profondeur d'un trajet donné, parmi les différents trajets, dans le tissu cible.

11. Procédé selon la revendication 10, dans lequel l'enregistrement d'un paramètre du flux sanguin comprend l'enregistrement dudit paramètre à une fréquence inférieure à 20 Hz.

12. Procédé selon la revendication 10, dans lequel l'enregistrement d'un paramètre du flux sanguin comprend l'enregistrement dudit paramètre à une fréquence comprise entre 20 Hz et 100 Hz.

13. Procédé selon la revendication 10, dans lequel l'enregistrement d'un paramètre du flux sanguin comprend l'enregistrement dudit paramètre à une fréquence supérieure à 100 Hz.

14. Procédé selon l'une des revendications 1 à 13, qui ne nécessite pas l'utilisation d'une source de lumière pulsée ni d'une source de lumière conçue pour générer une lumière modulée.
